# EUROPÄISCHE PATENTSCHRIFT

(11) **EP 0 401 230 B1**
(45) Veröffentlichungstag und Bekanntmachung des Hinweises auf die Patenterteilung: **06.10.1993**
(21) Anmeldenummer: 89901282.7
(22) Anmeldetag: 05.01.1989
(51) Int. Cl.: A61B 17/22

(54) **KATHETER FÜR DIE PERKUTANE GEFÄSS- UND ORGANCHIRURGIE MITTELS ENERGIESTRAHLUNG**
CATHETER FOR PERCUTANEOUS SURGERY OF BLOOD VESSELS AND ORGANS USING RADIANT ENERGY
CATHETER POUR LA CHIRURGIE PERCUTANEE DES VAISSEAUX ET DES ORGANES AU MOYEN D'UNE ENERGIE RAYONNEE

(30) Priorität: 08.02.1988 DE 3803697
(43) Veröffentlichungstag der Anmeldung: 12.12.1990
(73) Patentinhaber: Radtke, Wolfgang, Dr., 28329 Bremen (DE)
(72) Erfinder: Radtke, Wolfgang, Dr., 28329 Bremen (DE)
(74) Vertreter: Wolf, Eckhard, Dr.-Ing.
(86) Internationale Anmeldenummer: EP8900005
(87) Internationale Veröffentlichungsnummer: WO8906935

(56) Entgegenhaltungen:
- EP-A- 0 189 329
- FR-A- 2 317 903
- FR-A- 2 577 410
- GB-A- 2 020 557
- US-A- 3 397 699
- US-A- 3 568 659
- US-A- 4 627 436

## Beschreibung

Die Erfindung betrifft einen Katheter für die perkutane Gefäß- oder Organchirurgie mittels Energiestrahlung, wie Laser- oder Hochfrequenzstrahlung, vorzugsweise zur Durchführung perkutaner Valvotomie und zum Einschnitt membranöser Obstruktionen in Gefäßen und Organ-, insbesondere Herzhöhlen, mit mindestens einem Leiter zur Übertragung der Energiestrahlung von einer Energiequelle zu einer in der Nähe des distalen Katheterendes befindlichen Strahlungsaustrittsstelle, sowie mit einem mindestens zwei Drähte aufweisenden Positioniermechanismus, mit dem das distale Katheterende unter Freilassung einer Durchlaßöffnung in einem Gefäß oder in einer Organhöhle lösbar verankerbar ist, wobei die im wesentlichen in Katheterlängsrichtung verlaufenden Drähte im Bereich des distalen Katheterendes unter Bildung von radial über die Katheteroberfläche überstehenden konvexen Drahtbögen biegeelastisch aus einer gegen die Katheteroberfläche dicht anliegenden Grundposition abspreizbar sind.

Katheter dieser Art (EP-A 0 189 329) wurden bereits zur Rekanalisation blockierter Blutgefäße vorgeschlagen. Zu diesem Zweck wird der bekannte Katheter perkutan in ein Blutgefäß eingeführt und mit seinem distalen Ende durch das Gefäß bis zu der Stelle geschoben, an der ein Thrombus oder ein durch Arteriosklerose gebildeter Pfropfen das Gefäß blockiert. Anschließend werden die Drähte des Positioniermechanismus so weit abgespreizt, daß sie in geringer Entfernung vor dem Thrombus oder Pfropfen von innen her an der Gefäßwand anliegen. Der innerhalb des Katheters geführte Lichtleiter wird dabei im Bereich der Lichtaustrittsstelle im Blutgefäß zentriert und auf die Mitte des Thrombus oder Pfropfens ausgerichtet. Eine Positionierung des distalen Katheterendes an einer verengten Herzklappe oder im Bereich einer Verengung im Inneren einer Organhöhle ist damit jedoch nicht möglich.

Weiter wurde im Prioritätszeitraum vorgeschlagen, einen Katheter an seinem distalen Ende mit einem Positioniermechanismus in Form eines aufblasbaren Ballons zu verse hen (Nordstrom et al., "Laser Angioplasty: Controlled Delivery of Argon Laser Energy" Radiology 167, 1988, S. 463 - 465), mit dessen Hilfe die aus einem Lichtleiter austretende Laser-Strahlung im Gefäß zentriert oder gezielt abgelenkt werden kann. Durch den Ballon wird jedoch das Blutgefäß vollständig blockiert. Eine Verankerung des Katheters ist auch dort nur in relativ engen Blutgefäßen möglich, nicht jedoch in Organ-, insbesondere Herzhöhlen oder an Herzklappen.

Weiter wurde ein Katheter ohne Positioniermechanismus in Tierversuchen zum Einschneiden des septalen Herzmuskels eingesetzt (Isner et al. "The Current Status of Lasers in the Treatment of Cardiovascular Disease, IEEE Journal of Quantum Electronics, Nr. 12, 1984, S. 1406 - 1418). Der Katheter wurde dort unter Überwachung durch 2D-Echokardiographie perkutan ins Herz von Hunden eingeführt, wobei nach Kontakt der Lichtaustrittsstelle mit dem Herzmuskel der Einschnitt durchgeführt wurde. Damit war zwar nach dem Positionieren eine Vorhersage der Einschnittstelle möglich, nicht jedoch eine Positionierung der Lichtaustrittsstelle an einer vorbestimmten Stelle. Weiter wurde dort ein gezielter Einschnitt an einem menschlichen Herzen intraoperativ unter direkter Sicht durchgeführt.

Aus in-vitro-Versuchen mit postmortalem Gewebe und aus Tierversuchen ist es ferner bekannt, Katheter ohne Positioniermechanismus zur Entfernung einer membranösen Scheidewand in Herzhöhlen und einer Obstruktion großer Gefäße einzusetzan (Riemenschneider et al., "Laser Irradiation of Congenital Heart Disease: Potential for Palliation and Correction of Intracardiac and Intravascular Defects", American Heart Journal 106, Dez. 1983, S. 1389 -1393). Bei den in-vitro-Versuchen wurden die Katheter von außen her direkt in das Gefäßpräparat eingeführt, während bei den Tierversuchen mit einem perkutan eingeführten Katheter nur eine relativ ungezielte Perforation der Herzscheidewand durchgeführt werden konnte.

Der Erfindung liegt die Aufgabe zugrunde, einen Katheter der eingangs angegebenen Art zu entwickeln, der sich ohne Unterbrechung des Blutflusses und/oder der Organtätigkeit im Bereich eines nach dem Gefäß- oder Organhöhleninneren überstehenden Gefäß- oder Organteils bzw. einer durch eine Obstruktion gebildeten Verengung exakt positionieren läßt.

Zur Lösung dieser Aufgabe werden erfindungsgemäß die im Kennzeichenteil des Patentanspruchs 1 angegebenen Merkmale vorgeschlagen. Weitere vorteilhafte Ausgestaltungen und Weiterbildungen der Erfindung ergeben sich aus den abhängigen Ansprüchen.

Der Erfindung liegt der Gedanke zugrunde, den vorzugsweise vom proximalen Katheterende aus betätigbaren Positioniermechanismus des Katheters so auszubilden, daß sich die Drähte zugleich auf der distalen und auf der proximalen Seite einer durch ein Gefäß- bzw. Organteil oder durch eine Obstruktion gebildete Verengung abspreizen lassen, um so bei der perkutanen Organchirurgie die Voraussetzung für eine positionsgenaue Verankerung des distalen Katheterendes und damit für eine gezielte Ausrichtung der Energiestrahlung auf den für den Einschnitt vorgesehenen Wirkungsort zu schaffen. Dies wird erfindungsgemäß dadurch erreicht, daß die abgespreizten Drähte paarweise einander kreuzende, axial gegeneinander versetzt angeordnete Drahtbögen bilden, und daß sich im Kreuzungsbereich der Drahtbögen eine Rastvertiefung zur formschlüssigen Verankerung an einem nach dem Gefäß- oder Organhöhleninneren überstehenden Gefäß- oder Organteil bildet. Eine bevorzugte Ausgestaltung der Erfindung sieht vor, daß die Strahlungsaustrittsstelle durch Betätigung des Positioniermechanismus auf einen vorzugsweise im Bereich des Gefäß- oder Organteils befindlichen Wirkungsort ausrichtbar ist.

Um den Katheter zur Eröffnung stenotischer Herzklappen oder membranöser Stenosen einsetzen zu können, ist sein Positioniermechanismus gemäß einer bevorzugten Ausgestaltung der Erfindung bei ununterbrochener Klappentätigkeit auf dem freien Rand der Herzklappe verankerbar. Der Energiestrahlungsleiter wird dabei vorteilhaft EKG-getriggert, vorzugsweise bei geöffneter Herzklappe mit der Strahlungsenergie beaufschlagt. Um eine exakte Ausrichtung der Energiestrahlung auf die vorgesehene Einschnittstelle zu gewährleisten ist der Energiestrahlungsleiter zweckmäßig zumindest im Bereich der Strahlungsaustrittsstelle mit einem sich an dem Gefäß- oder Organteil abstützenden Draht des Positioniermechanismus verbunden, so daß die Strahlungsaustrittsstelle unmittelbar vor dem vorgesehenen Wirkungsort der Strahlung fixiert wird.

Gemäß einer bevorzugten Ausgestaltung der Erfindung weist der Positioniermechanismus mindestens zwei, vorzugsweise in gleichen Winkelabständen voneinander angeordnete Drahtpaare aus Metall oder Kunststoff auf, die sich beim Abspreizen in einer Herzklappe selbsttätig in die Winkel der fischmaul- oder dreieckförmigen Herzklappenöffnung legen.

Gemäß einer weiteren bevorzugten Ausgestaltung der Erfindung sind die Drähte eines jeden Drahtpaares im Bereich ihrer Bogenenden axial gegeneinander versetzt an zwei axial gegeneinander verschiebbaren, vorzugsweise koaxial zueinander angeordneten Katheterteilen verschiebefest angeordnet. Durch axiale Verschiebung der beiden Katheterteile am proximalen Katheterende lassen sich dann die Drahtpaare beim Einführen und Entfernen des Katheters an die Katheteroberfläche anlegen bzw. zur Fixierung des Katheters so weit abspreizen, daß die Einbuchtung im Kreuzungsbereich der Bögen z.B. am Rand einer Herzklappe anliegt und das distale Katheterende damit gegen Verschieben sichert.

Gemäß einer abgewandelten Ausgestaltung der Erfindung sind die Drähte relativ zum Katheter längsverschiebbar angeordnet und nur an ihren distalen Bogenenden verschiebefest mit dem Katheter verbunden.

Durch Verbinden zumindest eines der beiden Drähte, vorzugsweise des den proximalen Bogen bildenden Drahts eines jeden Drahtpaares mit einem parallel verlaufenden, vorzugsweise in einer gemeinsamen Ummantelung angeordneten Lichtleiter zur Übertragung von Laser-Strahlung sowie durch Anordnen der Austrittsstelle des Laserlichts im Bereich des sich beim Abspreizen bildenden Drahtbogens läßt sich z.B. zum Einschneiden einer Herzklappenverengung eine definierte räumliche Zuordnung zwischen der Lichtaustrittsstelle und dem in der Einbuchtung fixierten Klappenrand herstellen. Da zum einen die Lichtaustrittsstelle am Draht röntgenologisch oder sonographisch erkennbar ist und da zum anderen der Austrittswinkel der Laser-Strahlung bezüglich des mit dem Lichtleiter verbundenen Drahtes bekannt ist läßt sich die Richtung der austretenden Laser-Strahlung während des Eingriffs laufend kontrollieren. Um mehrere Einschnitte gleichzeitig oder nacheinander durchführen zu können, ohne den Katheter zu verdrehen, ist es zweckmäßig, mehrere oder alle Drähte mit Lichtleitern zu versehen.

Gemäß einer weiteren bevorzugten Ausgestaltung der Erfindung ist die Lichtaustrittsstelle im Bereich der sich beim Abspreizen der Drähte bildenden Einbuchtung, vorzugsweise proximal zur Kreuzungsstelle der Drahtbögen angeordnet. Die Lichtaustrittsstelle befindet sich damit in direktem Kontakt mit dem Wirkungsort der Laser-Strahlung auf dem in der Einbuchtung fixierten Gefäß- oder Organteil.

Gemäß einer weiteren bevorzugten Ausgestaltung der Erfindung ist die Abspreizhöhe der Drahtbögen einstellbar und vorzugsweise unter der Einwirkung einer Feder so nachführbar, daß die Drahtbögen mit dem einzuschneidenden Gefäß- oder Organteil in Kontakt bleiben. Durch selbsttätiges Vergrößern der Abspreizhöhe nach dem Einschneiden wird die Lichtaustrittsstelle zum Ende des Einschnitts nachgeführt, so daß kontrollierte Einschnitte unterschiedlicher Tiefe durchgeführt werden können.

Im folgenden wird die Erfindung anhand eines in der Zeichnung in schematischer Weise dargestellten bevorzugten Ausführungsbeispiels näher erläutert. Es zeigen
- Fig.1: eine Seitenansicht des proximalen Katheterendes in teilweise geschnittener Darstellung;
- Fig.2: eine Seitenansicht des distalen Katheterendes in teilweise geschnittener Darstellung mit vollständig (a) und teilweise (b) abgespreizten Drahtpaaren;
- Fig.3: eine Draufsicht auf das distale Katheterende mit anliegenden Drahtpaaren;
- Fig.4: einen Querschnitt durch den Katheter entlang der Linie A-A der Fig.1;
- Fig.5: einen Querschnitt durch den Katheter entlang der Linie B-B der Fig.1;
- Fig.6: einen Querschnitt durch den Katheter entlang der Linie C-C der Fig.2;
- Fig.7: einen mit einem Lichtleiter verbundenen Draht in geschnittener Darstellung.

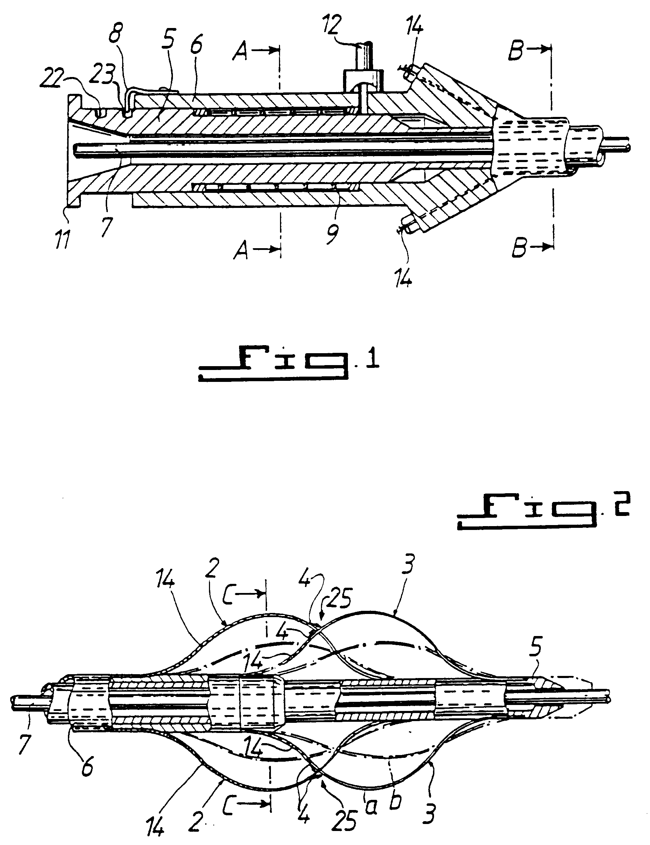

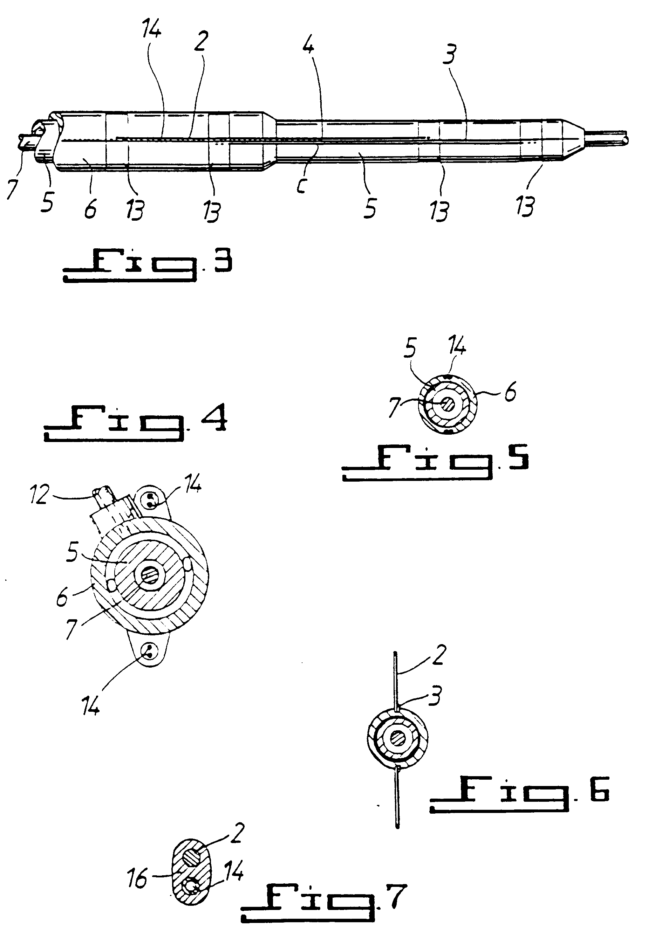

Der in der Zeichnung dargestellte Katheter weist zwei axial gegeneinander verschiebbare und koaxial zueinander angeordnete Katheterteile 5,6 auf. Das innere Katheterteil 5 ist am proximalen Ende mit einem Luer-Lock-Anschluß 11 versehen und weist ein in axialer Richtung durchgehendes Lumen 20 zur Durchführung eines Führungsdrahtes 7 auf, längs dessen der Katheter eingeführt werden kann. Ein am proximalen Ende des äußeren Katheterteils 6 angeordnetes Rastorgan 8 arretiert beim Eingreifen in die Rastvertiefungen 22,23 die beiden Teile 5 und 6 des Katheters gegeneinander. Die Rastvertiefungen 22,23 sind derart auf dem inneren Katheterteil 5 angeordnet, daß das Rastorgan 8 nur in den beiden Stellungen eingreift, die der gestreckten (c) und der maximal abgespreizten Position (a) der Drahtpaare entsprechen (Fig.2 und 3). Beim Herausziehen des Rastorgans 8 aus der Rastvertiefung 22 verschiebt die zwischen dem inneren 5 und dem äußeren Katheterteil 6 angeordnete Druckfeder 9 die beiden Katheterteile 5,6 in axialer Richtung so lange gegeneinander, bis die Drahtpaare 2,3 mit ihrer Einbuchtung 25 an dem einzuschneidenden Organteil oder der einzuschneidenden Obstruktion anliegen. Ein Schlauchanschluß 12 dient zum Spülen des Zwischenraumes zwischen dem inneren 5 und dem äußeren Katheterteil 6. Eine nicht gezeigte Axialführung verhindert das Verdrehen der beiden Katheterteile 5,6 gegeneinander. Je zwei von ihren Austrittsstellen an beiden Seiten des proximalen Katheterendes zu den Drahtpaaren 2,3 am distalen Katheterende führende ummantelte Lichtleiter 14 können einzeln oder getrennt über einen Konnektor an eine nicht gezeigte Laserlichtquelle angeschlossen werden.

Die aus Metall oder Kunststoff bestehenden Drähte 2 und 3 der beiden teilweise (b) bzw. vollständig (a) abgespreizten Drahtpaare des in Fig.2 dargestellten distalen Katheterendes kreuzen sich unter Bildung einer Einbuchtung 25, die zur Positionierung und Verankerung des Katheters dient. Die proximalen Enden der Drähte 2,3 sind axial gegeneinander versetzt mit dem äußeren Katheterteil 6 und die distalen Enden der Drähte 2,3 ebenfalls axial gegeneinander versetzt mit dem inneren Katheterteil 5 verbunden. Vier vom proximalen Katheterende kommende, in der Wand des äußeren Katheterteils 6 verlaufende Lichtleiter 14 treten zusammen mit den Drähten 2,3 aus der Oberfläche des äußeren Katheterteils 6 aus und erstrecken sich jeweils parallel zu den Drähten 2,3 bis zu den Lichtaustrittsstellen 4, wobei jeweils ein Lichtleiter 14 und ein Draht 2 bzw. 3 durch eine gemeinsame Ummantelung 16 verbunden sind (Fig.7). Die Lichtaustrittsstelle 4 ist derart ausgebildet, daß die durch kleine Pfeile angedeutete Laser-Strahlung tangential zu dem vom Draht gebildeten Bogen austritt (Fig.2). Die Verbindungsstellen der Drähte 2,3 mit dem inneren 5 bzw. äußeren Katheterteil 6 sind jeweils durch einen dünnen Metallring 13 verstärkt. In der in Fig.3 dargestellten Grundstellung der beiden Katheterteile 5 und 6 sind die Drahtpaare 2,3 gestreckt und liegen dicht an der Katheteroberfläche an. Das am proximalen Katheterende angeordnete Rastorgan 8 greift in dieser Position in die Rastvertiefung 22 ein.

Wird im Rahmen einer Untersuchung einer Herzklappe eine membranöse oder valvuläre Verengung festgestellt, so wird nach Sondierung der stenotischen Klappe ein Führungsdraht 7 so positioniert, daß sich der beschriebene Valvotomie-Katheter mit gestreckt an der Katheteroberfläche anliegenden Drähten 2,3 einführen läßt. Der Katheter wird derart in das Ostium der Klappe eingebracht, daß sich die Drahtpaare beim Abspreizen selbsttätig in die Winkel der fischmaul- oder dreieckförmigen Herzklappe legen, wobei der distale Draht 3 mit seiner Bogenmitte jenseits der Herzklappe und der proximale Draht 2 mit seiner Bogenmitte diesseits der Herzklappe zu liegen kommt. Nach Herausheben des Rastorgans 8 aus der Rastvertiefung 22 wird der innere Katheterteil 5 unter Aufspreizen der Drahtpaare durch die Druckfeder 9 in proximaler Richtung axial gegenüber dem äußeren Katheterteil 6 verschoben, bis der Rand der verengten Herzklappe in den Einbuchtungen 25 zu liegen kommt, die beim Abspreizen der Drahtpaare im Bereich der Kreuzungsstelle der Drähte 2,3 entstehen. Der Positionierungsvorgang wird ebenso wie die Ausrichtung der Lichtaustrittsstelle 4 röntgenologisch und mittels 2D-Echokardiographie verfolgt und kontrolliert. Nach der Positionierung des distalen Katheterendes im Bereich der zu trennenden Kommissuren liegen die Lichtaustrittsstellen 4 in direktem Kontakt an der vorgesehenen Einschnittstelle an, so daß nach Anschluß der Laser-Strahlungsquelle, z.B. einem Excimer-, Holmium-, oder Erbium-Laser, mit dem Einschnitt am Rand der verengten Herzklappe begonnen werden kann. Die Valvotomie wird durch EKG-getriggerte gepulste Laser-Strahlung jeweils bei geöffneter Herzklappe durchgeführt. Nach Herstellen eines ersten Einschnitts wird die Lichtaustrittsstelle 4 durch selbsttätiges Vergrößern der Abspreizhöhe zum Ende des Einschnitts nachgeführt, so daß kontrollierte Einschnitte unterschiedlicher Tiefe durchgeführt werden können. Die Einschnitt-Tiefe wird jeweils anhand vorab durchgeführter Messungen des Herzklappenrings bestimmt.

## Patentansprüche

1. Katheter für die perkutane Gefäß- oder Organchirurgie mittels Energiestrahlung, wie Laser- oder Hochfrequenzstrahlung, vorzugsweise zur Durchführung perkutaner Valvotomie und zum Einschnitt membranöser Obstruktionen in Gefäßen und Organ-, insbesondere Herzhöhlen, mit mindestens einem Leiter (14) zur Übertragung der Energiestrahlung von einer Energiequelle zu einer in der Nähe des distalen Katheterendes befindlichen Strahlungsaustrittsstelle (4), sowie mit einem mindestens zwei Drähte (2,3) aufweisenden Positioniermechanismus, mit dem das distale Katheterende unter Freilassung einer Durchlaßöffnung in einem Gefäß oder in einer Organhöhle lösbar verankerbar ist, wobei die im wesentlichen in Katheterlängsrichtung verlaufenden Drähte (2,3) im Bereich des distalen Katheterendes unter Bildung radial über die Katheteroberfläche überstehender konvexer Drahtbögen biegeelastisch aus einer gegen die Katheteroberfläche dicht anliegenden Grundposition abspreizbar sind, **dadurch gekennzeichnet**, daß die abgespreizten Drähte (2,3) paarweise einander kreuzende, axial gegeneinander versetzt angeordnete Drahtbögen bilden, und daß sich im Kreuzungsbereich der Drahtbögen eine Rastvertiefung (25) zur formschlüssigen Verankerung an einem nach dem Gefäß- oder Organhöhleninneren überstehenden Gefäß- oder Organteil bildet.

2. Katheter nach Anspruch 1, **dadurch gekennzeichnet**, daß die Strahlungsaustrittsstelle (4) durch Betätigung des Positioniermechanismus (2,3) auf einen vorzugsweise im Bereich des Gefäß- oder Organteils befindlichen Wirkungsort ausrichtbar ist.

3. Katheter nach Anspruch 1 oder 2, **dadurch gekennzeichnet**, daß der Energiestrahlungsleiter (14) zumindest im Bereich der Strahlungsaustrittsstelle (4) mit einem sich an dem Gefäß- oder Organteil abstützenden Draht des Positioniermechanismus (2,3) verbunden ist.

4. Katheter nach einem der Ansprüche 1 bis 3, **dadurch gekennzeichnet**, daß mindestens zwei, vorzugsweise in gleichen Winkelabständen voneinander angeordnete Drahtpaare (2,3) vorgesehen sind.

5. Katheter nach einem der Ansprüche 1 bis 4, **dadurch gekennzeichnet**, daß die Drähte (2,3) eines jeden Drahtpaares im Bereich ihrer Bogenenden axial gegeneinander versetzt an zwei axial gegeneinander verschiebbaren, vorzugsweise koaxial zueinander angeordneten Katheterteilen (5,6) verschiebefest fixiert sind.

6. Katheter nach einem der Ansprüche 1 bis 5, **dadurch gekennzeichnet**, daß die Drähte (2,3) relativ zum Katheter längsverschiebbar angeordnet und an ihren distalen Bogenenden verschiebefest mit dem Katheter verbunden sind.

7. Katheter nach einem der Ansprüche 1 bis 6, **dadurch gekennzeichnet**, daß zumindest einer der beiden Drähte (2,3), vorzugsweise der den proximalen Bogen bildende Draht (2) eines jeden Drahtpaares, mit einem parallel verlaufenden, vorzugsweise in einer gemeinsamen Ummantelung (16) angeordneten Lichtleiter (14) für Laser-Strahlung verbunden ist, und daß die Strahlungsaustrittsstelle (4) im Bereich des sich beim Abspreizen bildenden Drahtbogens angeordnet ist.

8. Katheter nach Anspruch 7, **dadurch gekennzeichnet**, daß jedem Draht (2,3) eines Drahtpaares ein Lichtleiter (14) zugeordnet ist.

9. Katheter nach Anspruch 7 oder 8, **dadurch gekennzeichnet**, daß die Strahlungsaustrittsstelle (4) im Bereich der sich beim Abspreizen der Drähte (2,3) bildenden Rastvertiefung (25), vorzugsweise proximal zur Kreuzungsstelle der Drahtbögen angeordnet ist.

10. Katheter nach einem der Ansprüche 1 bis 9, **dadurch gekennzeichnet**, daß die Abspreizhöhe einstellbar ist.

11. Katheter nach einem der Ansprüche 1 bis 10, **dadurch gekennzeichnet**, daß die Abspreizhöhe vorzugsweise unter der Einwirkung einer Feder (9) so nachführbar ist, daß die Drahtbögen mit dem zu behandelnden Gefäß- oder Organteil in Kontakt bleiben.

12. Katheter nach einem der Ansprüche 1 bis 11, **dadurch gekennzeichnet**, daß der Positioniermechanismus (2,3) an der Herzklappe eines schlagenden Herzens verankerbar ist und daß der Energiestrahlungsleiter (14) EKG-getriggert, vorzugsweise bei geöffneter Herzklappe, mit der Strahlungsenergie beaufschlagbar ist.

13. Katheter nach einem der Ansprüche 1 bis 12, **dadurch gekennzeichnet**, daß der Positioniermechanismus (2,3) vom proximalen Katheterende (11) aus betätigbar ist.

## Claims

1. Catheter for percutaneous surgery of blood vessels and organs using radiant energy such as laser or radiofrequency radiation, preferably for percutaneous valvotomy and for incision of membranous obstructions in vessels and hollow organs, especially in cardiac cavities containing at least one wave guide (14) for transmission of the energy radiation from its source to the point of emission of the energy radiation (4) close to the distal catheter end and also containing a positioning mechanism comprised of at least two wires (2,3) that allows reversable anchoring of the distal catheter end in a vessel or hollow organ leaving a throughput opening, wherein the wires (2,3) which are mostly running parallel to the axis of the catheter and can be spread at the distal catheter end from a ground position flat and closely adherent to the catheter surface to form two convex elastically flexible wire arches radially extending over the catheter surface, characterized that the spread wires (2,3) form pairs of wire arches axially displaced against each other and intersecting each other and that an indentation (25) forms at the intersection of the wire arches that allows form fitting anchoring on a vessel or organ part protruding into the lumen of the vessel or hollow organ.

2. Catheter according to claim 1, characterised in that by means of the positioning mechanism (2,3) the point of emission of the energy radiation (4) can be aimed at the target preferably located in the vessel or organ part.

3. Catheter according to claim 1 or 2, characterised in that the waveguide for the energy radiation (14) is at least in the region of the point of emission (4) combined with the wire arch of the positioning mechanism (2,3) that anchors on the vessel or organ part.

4. Catheter according to one of claims 1 to 3 comprising of at least two wire pairs (2,3) preferably arranged at equal angles to each other.

5. Catheter according to one of claims 1 to 4, characterised in that the wires (2,3) of each wire pair are axially displaced against each other at their arch ends and firmly attached to two catheter components (5,6) that are preferably arranged coaxially to each other and axially movable against each other.

6. Catheter according to one of claims 1 to 5, characterised in that the wires (2,3) are axially movable relative to the catheter and at the distal end of the arches firmly attached to the catheter.

7. Catheter according to one of claims 1 to 6, characterised in that at least one of both wires (2,3), preferably the wire forming the proximal arch (2) of each wire pair carries a waveguide for the energy radiation (14) running parallel and preferably enclosed in a common coating (16) and wherein the point of emission of the energy radiation (4) is located in the region of the wire arch that forms during spreading.

8. Catheter according to claim 7, characterised in that each wire (2,3) of each wire pair carries a waveguide (14).

9. Catheter according to claim 7 or 8, characterised in that the point of emission (4) is located close to the indentation (25) forming when the wires (2,3) are spread, and preferably proximal to the intersection of the wire arches.

10. Catheter according to one of claims 1 to 9, characterised in that the degree of spreading is variable.

11. Catheter according to one of claims 1 to 10, characterised in that the degree of spreading is adjustable, preferably by means of a spring (9) so that the wire arches remain in contact with the vessel or organ part to be treated.

12. Catheter according to one of claims 1 to 11, characterised in that the positioning mechanism (2,3) can be anchored on the valve of a beating heart and the energy radiation can be launched into the waveguide (14) by EKG trigger, preferably while the cardiac valve is open.

13. Catheter according to one of claims 1 to 12, characterised in that the positioning mechanism (2,3) can be handled from the proximal catheter end (11).

## Revendications

1. Cathéter pour la chirurgie percutanée de vaisseaux ou d'organes au moyen de rayonnement énergétique tel que rayonnement laser ou rayonnement à haute fréquence, de préférence pour l'exécution de valvotomie percutanée et pour l'incision d'obstructions membraneuses dans des vaisseaux et des cavités d'organes, en particulier du coeur, comportant au moins un conducteur (14) pour la transmission du rayonnement énergétique d'une source d'énergie à un point de sortie de rayonnement (4) situé à proximité de l'extrémité distale du cathéter, et un mécanisme de positionnement présentant au moins deux fils (2, 3) avec lequel l'extrémité distale du cathéter peut être ancrée dans un vaisseau ou dans une cavité d'organe en laissant libre une ouverture de passage, les fils (2, 3), s'étendant de manière générale dans la direction longitudinale du cathéter, pouvant, dans la zone de l'extrémité distale du cathéter, être écartés élastiquement par flexion d'une position de base, où ils sont appliqués contre la surface du cathéter, avec formation d'arcs de fil convexes saillant radialement de cette surface, caractérisé par le fait que les fils (2, 3) écartés forment des arcs décalés axialement et se croisant deux à deux, et que dans la zone de croisement des arcs de fil se forme un creux d'engagement (25) pour l'ancrage par emboîtement à une partie de vaisseau ou d'organe saillant vers l'intérieur du vaisseau ou de la cavité d'organe.

2. Cathéter selon la revendication 1, caractérisé par le fait que le point de sortie de rayonnement (4) peut, par actionnement du mécanisme de positionnement (2, 3), être orienté vers un endroit d'action situé de préférence dans la zone de la partie de vaisseau ou d'organe.

3. Cathéter selon l'une des revendications 1 et 2, caractérisé par le fait que le conducteur de rayonnement énergétique (14) est, au moins dans la zone du point de sortie de rayonnement (4), joint à un fil du mécanisme de positionnement (2, 3) s'appuyant sur la partie de vaisseau ou d'organe.

4. Cathéter selon l'une des revendications 1 à 3, caractérisé par le fait qu'il est prévu au moins deux paires de fils (2, 3) placées de préférence à des intervalles angulaires égaux.

5. Cathéter selon l'une des revendications 1 à 4, caractérisé par le fait que les fils (2, 3) de chaque paire de fils sont fixés dans la zone de leurs extrémités d'arc, décalés axialement l'un de l'autre, à deux parties (5, 6) du cathéter mobiles axialement l'une par rapport a l'autre et de préférence placées coaxialement.

6. Cathéter selon l'une des revendications 1 à 5, caractérisé par le fait que les fils (2, 3) sont déplaçables longitudinalement par rapport au cathéter et joints de manière fixe à celui-ci à leur extrémité d'arc distale.

7. Cathéter selon l'une des revendications 1 à 6, caractérisé par le fait qu'au moins un des deux fils (2, 3), de préférence le fil (2) formant l'arc proximal, de chaque paire de fils est joint à un conducteur de lumière (14) pour rayonnement laser s'étendant parallèlement a lui et de préférence placé avec lui dans une gaine commune (16), et que le point de sortie de rayonnement (4) est placé dans la zone de l'arc de fil se formant lors de l'écartement.

8. Cathéter selon la revendication 7, caractérisé par le fait qu'à chaque fil (2, 3) d'une paire de fils est adjoint un conducteur de lumière (14).

9. Cathéter selon l'une des revendications 7 et 8, caractérisé par le fait que le point de sortie de rayonnement (4) est placé dans la zone du creux d'engagement (25) se formant lors de l'écartement des fils (2, 3), de préférence proximalement par rapport au point de croisement des arcs de fil.

10. Cathéter selon l'une des revendications 1 à 9, caractérisé par le fait que la hauteur d'écartement est réglable.

11. Cathéter selon l'une des revendications 1 à 10, caractérisé par le fait que la hauteur d'écartement peut être conduite de préférence sous l'action d'un ressort (9) de façon que les arcs de fil restent en contact avec la partie de vaisseau ou d'organe à traiter.

12. Cathéter selon l'une des revendications 1 à 11, caractérisé par le fait que le mécanisme de positionnement (2, 3) peut être ancré à une valvule d'un coeur qui bat, et que le conducteur de rayonnement énergétique (14) peut être alimenté en énergie rayonnante avec déclenchement par ECG, de préférence lorsque la valvule est ouverte.

13. Cathéter selon l'une des revendications 1 à 12, caractérisé par le fait que le mécanisme de positionnement (2, 3) peut être actionné depuis l'extrémité proximale (11) du cathéter.
